## Europäisches Patentamt
## European Patent Office
## Office européen des brevets

(19)

(11) Numéro de publication: **0 192 576**
**B1**

(12) **FASCICULE DE BREVET EUROPEEN**

(45) Date de publication du fascicule du brevet:
23.11.89

(51) Int. Cl.⁴: **A61M 25/00, A61B 1/00**

(21) Numéro de dépôt: 86400347.0

(22) Date de dépôt: **19.02.86**

(54) Sonde endovasculaire orientable.

(30) Priorité: **22.02.85 FR 8502568**

(43) Date de publication de la demande:
**27.08.86 Bulletin 86/35**

(45) Mention de la délivrance du brevet:
**23.11.89 Bulletin 89/47**

(84) Etats contractants désignés:
**AT BE CH DE FR GB IT LI LU NL SE**

(56) Documents cités:
**US-A- 3 665 928**
**US-A- 3 773 034**
**US-A- 4 403 985**

(73) Titulaire: **MEDICORP RESEARCH LABORATORIES CORPORATION, 1200 North Federal Highway Suite 200-26, Boca Raton Florida 33432(US)**

(72) Inventeur: **Karcher, Gilles, 2, Rue Lafayette, F-54000 Nancy(FR)**
Inventeur: **Amor, Max, 9, Square de Liège, F-54500 Vandoeuvre(FR)**
Inventeur: **Niddam, Roger, 43, Allée du Jardin Anglais, F-93340 Le Rancy(FR)**
Inventeur: **Villemot, Jean-Pierre, Le Trident Rue Cyffle, F-54000 Nancy(FR)**

(74) Mandataire: **Laget, Jean-Loup, Cabinet Pierre Loyer 77, rue Boissière, F-75116 Paris(FR)**

ACTORUM AG

## Description

L'invention concerne une sonde endovasculaire orientable.

Pour l'exploration des artères, on utilise fréquemment des sondes souples qui sont poussées de l'extérieur le long d'un guide métallique. La forme de ces sondes et leur souplesse sont adaptées aux vaisseaux à explorer, mais il reste généralement difficile de faire franchir à la sonde les coudes ou les bifurcations.

Par ailleurs, il existe des sondes spécialisées qui ont une forme particulière, pour l'exploitation de certains vaisseaux comme les artères coronaires. Parmi ces sondes, on peut citer les sondes de type Judkins, Bourrassa, Amplatz, Cobra, ou autres. Pour chacun de ces types de sonde, il existe une famille de cinq ou six modèles pour s'adapter aux différentes grosseurs d'organes, par exemple, ou à certains types de déformations des organes. Ces familles de sondes sont caractérisées par une même allure générale, mais par des courbures différentes au niveau d'un coude par exemple.

Le but de l'invention est de proposer une sonde dont l'extrémité soit orientable depuis l'extérieur afin de permettre tout à la fois de faire fanchir facilement à ladite sonde les coudes et de réduire notablement le nombre des modèles de sonde de chaque famille.

Il est connu d'utiliser pour des examens de l'appareil digestif une sonde dont la tête est articulée sur le corps par une zone formée d'un soufflet en deux parties. Des canaux de circulation de fluide sont prévus dans le corps, afin que l'envoi d'un fluide sous pression à l'entrée du circuit crée une circulation alternativement dans chaque partie du soufflet. La tête de la sonde est ainsi amenée à osciller par rapport au corps de façon définie et non modifiable.

Une telle disposition qui permet de faire progresser la sonde par elle-même dans l'appareil digestif n'est pas directement applicable à une sonde objet de l'invention.

Selon l'invention, l'orientation de la tête de la sonde est modifiable à tout moment et il est prévu à cet effet des moyens de commande d'orientation particulièrement souples.

L'invention a pour objet une sonde endovasculaire orientable, comportant un corps de sonde et une tête de sonde et présentant entre la tête et le corps une zone d'articulation, dans laquelle est prévu au moins un soufflet susceptible d'être gonflé par un fluide pour provoquer l'orientation de la tête dans une direction radiale opposée au soufflet gonflé, caractérisée en ce que chaque soufflet s'étend sur moins de la moitié de la circonférence de la sonde et que pour chaque soufflet un canal d'alimentation en fluide logé le long du corps de la sonde est prévu.

Selon d'autres caractéristiques de l'invention:

– les soufflets sont alimentés en fluide par des canaux logés le long du corps de la sonde;
– la sonde comporte deux soufflets placés de part et d'autre du corps de sonde pour assurer l'orientation de la tête de sonde dans deux directions opposées,

– la sonde comporte quatre soufflets placés deux-à-deux de part et d'autre du corps de sonde, pour assurer l'orientation de la tête de sonde dans quatre directions et dans les bissectrices de ces directions;
– la sonde présente une courbure et comporte un seul soufflet disposé à l'extérieur de la zone de courbure pour faire varier cette courbure.

D'autres caractéristiques ressortent de la description suivante faite avec référence au dessin annexé sur lequel on peut voir:

Figure 1, une vue en coupe longitudinale d'un exemple de réalisation d'une sonde orientable selon l'invention;
Figure 2, une vue en coupe transversale selon la ligne A-A de la figure 1;
Figure 3, une vue en coupe transversale d'un exemple de réalisation d'une sonde orientable dans plusieurs directions.
Figure 4, une vue schématique dans le plan axial moyen d'une sonde de forme particulière dont la courbure est variable par commande du gonflage d'un soufflet, selon l'invention.

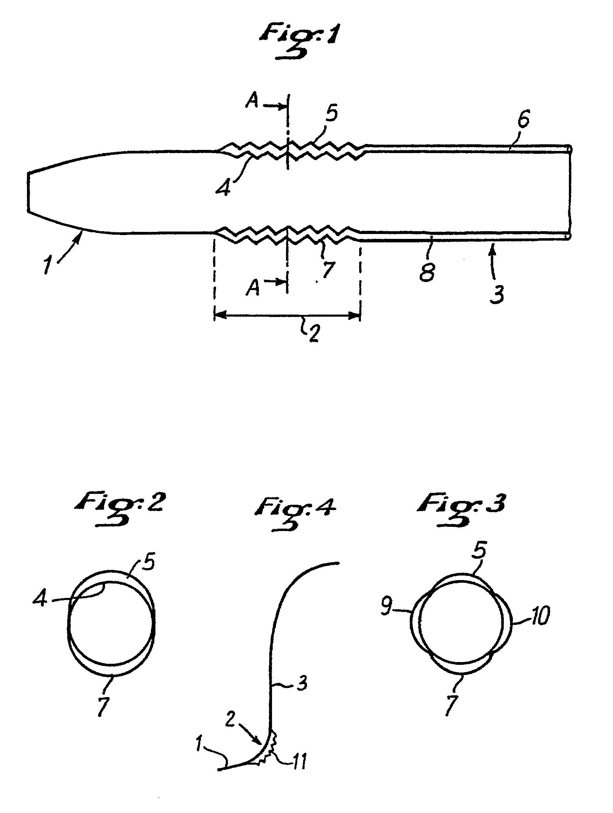

En se reportant à la figure 1, on voit que la sonde endovasculaire présente une tête 1 de forme classique et, entre cette tête et le corps 3 de la sonde, une zone d'articulation 2. Dans cette zone 2, la paroi 4 de la sonde est extensible et à l'extérieur de cette paroi est disposé un soufflet 5 susceptible d'être rempli de fluide, au moyen d'un canal latéral 6 courant le long de la sonde. Ce soufflet 5 (figure 2) s'étend sur une certaine largeur, pouvant aller jusqu'à la moitié de la circonférence de la sonde. Un soufflet 7 symétrique du premier par rapport au plan axial horizontal est alimenté en fluide par un canal latéral 8.

Lorsque la sonde est dans un vaisseau et devant une birfucation par exemple, on peut orienter la tête 1 en injectant du fluide dans l'un des canaux 6 ou 8. Sous l'action de la pression de ce fluide, le soufflet correspondant, 5 ou 7 respectivement, se gonfle et s'étend, faisant ainsi basculer la tête 1 de sonde vers le bas ou vers le haut, respectivement.

Dans l'exemple de réalisation de la figure 3, la sonde est munie, outre les deux soufflets 5 et 7 supérieur et inférieur, de deux autres soufflets 9 et 10 latéraux. Lorsqu'un des soufflets 9 ou 10 est gonflé par un fluide la tête de sonde 1 s'oriente du côté opposé à ce soufflet. On peut ainsi orienter la tête 1 dans quatre directions en gonflant un seul des quatre soufflets à la fois. On peut également, en gonflant simultanément deux soufflets adjacents comme 5 et 9, orienter la tête de sonde dans la direction comprise entre les soufflets 7 et 10, c'est-à-dire vers la gauche, mais à 45° de la verticale, dans la bissectrice des directions d'orientation correspondant aux deux soufflets.

Dans l'exemple de réalisation de la figure 4, une sonde de type particulier, par exemple pour l'exploration d'une artère coronaire, présente une tête 1, un corps 3, et entre les deux une zone d'articulation 2 qui est courbe à l'état de repos. A l'extérieur de cette zone d'articulation, la sonde présente un soufflet gonflable 11. Suivant le degré de gonflage de ce soufflet 11, la courbure de la sonde est modifiée. On peut ainsi n'utiliser qu'un seul modèle de sonde et l'adapter au cas clinique par simple réglage du gonflage du soufflet. Un avantage de cette disposition est de permettre une réduction du temps de recherche des artères coronaires par exemple.

Ainsi, depuis l'extérieur et par simple injection de pression dans l'un ou dans deux des canaux latéraux, on peut imposer à la tête de sonde une orientation facilitant sa progression. Le diamètre et la position de la zone d'articulation sont déterminés en fonction de la nature du cathétérisme et des artères à aborder.

**Revendications**

1. Sonde endovasculaire orientable, comportant un corps (3) de sonde et une tête (1) de sonde et présentant entre la tête (1) et le corps (3) une zone d'articulation (2), dans laquelle est prévu au moins un soufflet (5, 7, 9, 10) susceptible d'être gonflé par un fluide pour provoquer l'orientation de la tête (1) dans une direction radiale opposée au soufflet gonflé, caractérisée en ce que chaque soufflet (5, 7, 9, 10) s'étend sur moins de la moitié de la circonférence de la sonde et que pour chaque soufflet (5, 7, 9, 10) un canal (6, 8) d'alimentation en fluide logé le long du corps (1) de la sonde est prévu.

2. Sonde selon la revendication 1, caractérisée en ce que dans la zone d'articulation (2), la paroi (4) de la sonde est extensible.

3. Sonde selon la revendication 1, caractérisée en ce que la sonde comporte deux soufflets (5, 7) placés de part et d'autre du corps de sonde pour assurer l'orientation de la tête de sonde dans deux directions opposées.

4. Sonde selon la revendication 1, caractérisée en ce que la sonde comporte quatre soufflets (5, 7, 9, 10) placés deux-à-deux de part et d'autre du corps de sonde, pour assurer l'orientation de la tête de sonde dans quatre directions et dans les bissectrices de ces directions.

5. Sonde selon la revendication 1, caractérisée en ce que la sonde présente une courbure et comporte un soufflet disposé à l'extérieur de la zone de courbure pour faire varier cette courbure.

**Claims**

1. Rotatable endovascular catheter, consisting of catheter body (3) and a catheter head (1) and having between the head (1) and the body (3) an articulation zone (2) in which at least one sac or pocket (5, 7, 9, 10) is provided which can be inflated by a fluid so as to cause the head (1) to rotate in a radial direction away from the inflated sac, characterised in that each sac (5, 7, 9, 10) extends over less than half the circumference of the catheter and in that for each sac (5, 7, 9, 10) a supply channel (6, 8) for feeding the fluid is provided, being placed along the body (1) of the catheter.

2. Catheter according to Claim 1, characterised in that within the zone (2) of articulation the inner surface or wall (4) of the catheter is extensible.

3. Catheter according to Claim 1, characterised in that the catheter comprises two sacs (5, 7) placed on either side of the body of said catheter in order to enable the catheter head to be rotated in two opposing directions.

4. Catheter according to Claim 1, characterised in that the catheter comprises four sacs (5, 7, 9, 10) placed in pairs on either side of the catheter body in order to enable the head of said catheter to be rotated in four directions and within the bisecting lines of these directions.

5. Catheter according to Claim 1, characterised in that the catheter is curved and comprises a sac placed on the exterior of the zone of curvature so as to allow said curve to be varied.

**Patentansprüche**

1. Verstellbare endovaskuläre Sonde mit einem Sondenkörper (3) und einem Sondenkopf (1), wobei sich zwischen dem Sondenkopf (1) und dem Sondenkörper (3) eine Schwenkzone (2) befindet, in der mindestens ein Balgteil (5, 7, 9, 10) vorgesehen ist, das von einem Fluid aufgeblasen werden kann, um die Ausrichtung des Sondenkopfs (1) in eine dem aufgeblasenen Balgteil radial entgegengesetzte Richtung herbeizuführen, dadurch gekennzeichnet, daß jedes Balgteil (5, 7, 9, 10) sich über weniger als den halben Umfang der Sonde erstreckt, und daß für jedes Balgteil (5, 7, 9, 10) ein längs des Sondenkörpers (1) angeordneter Fluidzuführungskanal (6, 8) vorgesehen ist.

2. Sonde nach Anspruch 1, dadurch gekennzeichnet, daß die Sondenwand (4) in der Schwenkzone (2) dehnbar ist.

3. Sonde nach Anspruch 1, dadurch gekennzeichnet, daß die Sonde zwei Balgteile (5, 7) aufweist, von denen jeweils einer auf einer der beiden Seiten des Sondenkörpers angeordnet ist, um das Verstellen des Sondenkopfs in zwei zueinander entgegengesetzten Richtungen zu ermöglichen.

4. Sonde nach Anspruch 1, dadurch gekennzeichnet, daß die Sonde vier Balgteile (5, 7, 9, 10) aufweist, die paarweise beiderseits des Sondenkörpers angeordnet sind, um das Verstellen des Sondenkopfs in vier Richtungen und in Richtung der Winkelhalbierenden zwischen diesen Richtungen zu ermöglichen.

5. Sonde nach Anspruch 1, dadurch gekennzeichnet, daß die Sonde eine Biegung aufweist und ein Balgteil besitzt, das an der Außenseite des Bereichs der Biegung angeordnet ist, um eine Änderung dieser Biegung vornehmen zu können.

*Fig. 1*

*Fig. 2*   *Fig. 4*   *Fig. 3*